# EUROPEAN PATENT SPECIFICATION

(11) **EP 1 585 585 B1**
(45) Date of publication and mention of the grant of the patent: **04.04.2012**
(21) Application number: 03748117.3
(22) Date of filing: 06.10.2003
(51) Int. Cl.: A61K 9/16, B01D 9/00, B01J 2/06, B01F 13/00, B01J 19/00

(54) **PROCESS AND REACTOR FOR THE MANUFACTURE OF POWDERS OF INHALABLE MEDICAMENTS**
VERFAHREN UND REAKTOR ZUR HERSTELLUNG VON PULVERN FÜR INHALATIVE MEDIKAMENTE
PROCEDE DE FABRICATION DE POUDRES POUR MEDICAMENTS POUVANT INHALES

(30) Priority: 17.10.2002 EP 02023273
(43) Date of publication of application: 19.10.2005
(73) Proprietor: Boehringer Ingelheim Pharma GmbH & Co.KG, 55216 Ingelheim am Rhein (DE); ECOLE POLYTECHNIQUE FEDERALE DE LAUSANNE (EPFL), 1015 Lausanne (CH)
(72) Inventor: JONGEN, Nathalie, CH-1028 PREVERENGES (CH); LEMAITRE, Jacques, CH-1012 LAUSANNE (CH); BOWEN, Paul, CH-1260 NYON (CH); DONNET, Marcel, CH-1033 CHESEAUX (CH); SCHIEWE, Jörg, 55129 MAINZ (DE); ZIERENBERG, Bernd, 55411 BINGEN (DE); SOARE, Lucica, Cristina, CH-1004 LAUSANNE (CH)
(86) International application number: PCT/EP2003/011010
(87) International publication number: WO 2004/034943

(56) References cited:
- WO-A-00/38811
- WO-A-02/00200
- WO-A-98/02237
- WO-A-02/089942
- DE-B- 1 262 240

## Description

### BACKGROUND OF THE INVENTION

### 1. TECHNICAL FIELD

The invention relates to an improved process for the production or powders organic compounds by precipitation from liquid mixtures.

### 2. BACKGROUND INFORMATION

The international patent application WO 98/2237 discloses a process for the production of inorganic powders by precipitation from a liquid reaction mixture, the method comprising passing along a tubular reactor a segmented reaction flow comprised of discrete volumes of the reaction mixture separated by discrete volumes of a separating fluid which is substantially immiscible with said reaction mixture, the residence time of said discrete volumes of reaction mixture in the reactor being sufficient for the precipitation reaction to be effected.

Unfortunately, this process is not applicable for inhalable medicaments.

For inhalable medicaments a well-defined size and shape of the crystals is a pre-requisite. Inhalatives require a certain form of the medicament. For example, micronised medicaments or active ingredients generally come in solid form. In order to guarantee the inhalability of the medicament, high requirements are placed on the particle size, the particle size distribution, the morphology, the stability and the flow performance.

In general, the entire administered dose of the medicament does not reach the lungs, rather only a part of this does. The particle size has a substantial influence on the proportion of the medicament which actually reaches the lungs. For this reason, particles are preferred which have a diameter of less than 20 µm, preferably less than 5 µm and greater than 0.3 µm. The diameter of the particle should be within the given window and furthermore should have the narrowest possible size distribution. Larger particles are separated off during respiration in the upper airways whilst smaller particles are not deposited in the lungs and these leave again when exhaling.

Therefore, there is a great requirement for processes which achieve powders of inhalable medicaments with uniform shape, small size and narrow size distribution.

It is known that crystallization of drug actives can be ultrasonically promoted, e.g. Causland and Cains in Drug Delivery systems & sciences, volume 2 no 2, June/ July 2002, pp 47-51.

However, there is no hint that the application of ultrasound to a tubular reactor with a segmented reaction flow would yield such a desired crystal formation.

### BRIEF SUMMARY OF THE INVENTION

It has now be found surprisingly, that the application of ultrasound to a tubular reactor with a segmented reaction flow achieves crystals of inhalable medicaments with the desired shape and size.

Therefore, the invention relates to an improved process for the production of powders of inhalable medicaments by crystallization from a supersaturated fluid containing said medicament, the method comprising passing along a tubular reactor
(a) a segmented flow of that fluid comprised of discrete volumes; or
(b) a fluid mixture being separated by discrete volumes of a separating fluid which is substantially immiscible with said fluid,
characterized in that the crystallization is initiated by application of ultrasound.

A second embodiment of the present invention is a micro-reactor for implementing the process according to this invention comprising a micro-mixer, a segmenter and a tubular reactor, wherein
- the dimensions of the micro-mixer for dividing the added fluids which are to be mixed is in the range of 10 µm to 1 mm, preferably between 25 µm to 200 µm,
- the dimensions of the channels of the segmenter lie in the range of 0.1 to 5 mm, preferably in the range of between 0.2 mm and 5 mm, and
the tubular reactor is configured to be tube-, pipe- or channel-shaped with diameters of the channels in the range of 0.5 to 10 mm, preferably 1 mm to 2 mm, and with a length of between 10 cm and 200 m, preferably between 1 m and 25 m and is equipped with an external ultrasound source.

Furthermore the invention relates to an inhalable medicament with an aerodynamic diameter of less than 20 µm, preferably less than 5 µm and greater than 0.3 µm, characterized in that it is produced by means of the inventive process.

### BRIEF DESCRIPTION OF THE DRAWINGS

Figure 1 shows a schematic flow chart of fenoterol crystallization.
Figure 2 shows the X-Ray diffractogram of dried sample of fenoterol (SFTR-13.06.02) Tween (Polysorbate) and the reference powder.
Figure 3 shows the SEM image of dried material of fenoterol (SFTR-13.06.02) Tween
Figure 4 shows a schematic flow chart of Budesonide (11.07.02-SFTR) crystallization
Figure 5 shows the X-rays diffractogram of budesonide powder crystallised and reference material.

### DETAILED DESCRIPTION OF THE INVENTION

The invention preferably relates to a process wherein the segmented flow passes along the tubular reactor as a plug flow.

Furthermore preferred is a process wherein the tubular reactor consists of the following segments:
(i) a residence time (t_{R}) segment;
(ii) an ultrasound time (t_{US}) segment, in particular wherein t_{US} is 1 to 30 s or wherein t_{US} is 0.5 to 15 min and t_{A} is 0 to 30 s; and
(iii) optionally an aging time (t_{A}) segment.

The particle size distribution of the organic compounds can be fine-tuned depending on the ratio of t_{R}, t_{US} and t_{A}. Smaller particle size distributions can be obtained if longer t_{US} are applied.

Preferably an ultrasound with a frequency of 20 to 60 kHz and/or an energy density from 10 to 80 WL⁻¹is applied.

Another preferred embodiment is a process wherein the segmented flow or a precursor segmented flow from which the segmented flow is subsequently generated. is produced by passing the fluid containing the organic compound or a component thereof and the separating fluid to a chamber having a restricted outlet from which the segmented flow issues, in particular wherein the segmented flow is produced in a segmentation arrangement comprised of two concentric tubes, said chamber being provided at the outlet of the inner of the tubes and said chamber has an internal diameter of 2 mm to 10 mm.

Preferably, the innermost tube has an internal diameter of 0.1 to 2 mm and/or the distance between the outlet of the innermost tube and the inlet of the restriction is in the range 0. 1 to 5 mm.

Preferably the separating fluid is passed to said chamber along the innermost tube.

Furthermore preferred is a process wherein the segmented flow is prepared by passing the fluid containing the organic compound and the separating fluid to said chamber thereby producing the segmented reaction flow, in particular wherein discrete volumes of said component of the fluid comprising the organic compound are separated by discrete volumes of the separating fluid and the segmented reaction flow is produced by admixing said discrete volumes of the fluid containing said organic compound with the remaining component(s) of the mixture.

Another preferred embodiment is a process wherein the segmented reaction flow is prepared from said precursor flow by injecting said latter flow and the further component(s) of the fluid containing the medicament to a chamber having a restricted outlet under conditions such that said further component(s) of the reaction mixture become admixed with the discrete volumes of said first component of the reaction mixture whereby the segmented reaction flow is produced, in particular wherein the segmented reaction flow is produced in a mixing arrangement, in particular wherein the chamber of the mixing arrangement has a diameter of 9 mm to 10 mm, having preferably an internal diameter of 0.1 to 2 mm, comprised of two concentric tubes said chamber being provided at the outlet of the inner of the two tubes; and/or wherein the distance between the outlet of the innermost tube of the mixing arrangement and the inlet of the restriction is in the range 0.1 to 5 mm.

Furthermore preferred is a process wherein a fluid mixture containing the medicament is prepared in a micro-mixer before the segmentation, in particular wherein the fluid mixture is a mixture of a solution of the medicament with a suitable precipitant to create a meta-stable supersaturated fluid.

Another preferred embodiment is a process wherein the fluid mixture is a mixture of a solution of the medicament with a suitable detergent in order to influence particle size and shape during the subsequent crystallization process.

Preferably the separating fluid is
a hydrocarbon, in the event that the organic compound is water-soluble, in particular a C₆₋₁₈ hydrocarbon; or
a lower alcohol or water, in the event that the organic compound is insoluble in water.

In the following text, examples are listed for the active ingredients, the adjuvants, the solvent and the precipitation agent.

The following are used as medicaments or active ingredients:
- as anticholinergics; ipratropiumbromide, oxitropium, tiotropiumbromide, tiotropriumbromide-monohydrate,
- as betasympathomimetics: bambuterol, biolterol, carbuterol, formoterol, clenbuterol, fenoterol, hexoprenalin, procaterol, ibuterol, pirbuterol, tulobuterol, reproterol, salbutamol, salmeterol, sulfonterol, terbutalin, orciprenalin, 1-(2-fluoro-4-hydroxy-phenyl)-2-[4-(1-benzimidazolyl)-2-methyl-2-butylamino]ethanol,erythro-5'-hydroXy-8'-(1-hydroxy-2-isopropylaminobutyl)-2H-1,4-benzoxazin-3-(4H)-one, 1-(4-amino-3-chloro-5-trifluoromethyl-phenyl)-2-tert.-butyl-amino)ethanol, 1-(4-ethoxycarbonylamino-3-cyano-5-fluorophenyl)-2-(tert.-butylamino)ethanol,
- as antiallergics: disodiumchromeglicate, nedocromil, epinastin, and
- as steroids: flunisolide, dexamethasone-21-isonicotinate, seratrodast, mycophenolate mofetil, pranlukast, zileuton, butixocort, budesonide, deflazacort, fluticasone, proedrol, mometasin furoate, tipredan, beclometasone (or the 16,21-dipropionate), beclomethasone, Douglas, icomethasone enbutate, cyclometasone, cloprednol, fluocortin butyl, halometasone, deflazacort, alclometasone, cyclometasone, alisactide, prednicarbate, hydrocortisone-butyratepropionate, tixocortolpivalate, alclometaszone-dipropionate, lotrisone, canesten-HC, deprodone, fluticasone-propionate, methylprednisolone-aceponate, halopredone-acetate, mometasone, mometasone-furoate, hydrocortisone-aceponate, mometasone, ulobetasol-propionate, aminogluethimide, triamciolone, hydrocortisone, meprednisone, fluorometholone, dexamethasone, betamethasone, medrysone fluclorolone acetonide, fluocinolone acetonide, paramethasone-acetate, deprodon propionate, aristocort-diacetate, fluocinonide, mazipredone, difluprednate, betamethasone valerate, dexamethasoneisonicotinate, beclomethasone-dipropionate, fluocortoloncapronate, formocortal, triamcinolon-hexacetonide, cloprednol, formebolone, clobetasone, endrisone, flunisolide, halcinonide, fluazacort, clobetasol, hydrocortisone-17-butyrate, diflorasone, fluocortin, amcinonide, netamethasone dipropionate, cortivazole, betamethasoneadamantoate, fluodexane, trilostan, budesonide, clobetasone, demetex, trimacinolone benetonide, 9.alpha.-chloro-6.alpha.-fluoro-11.beta.17.alpha.-dihydroxy-16.-alpha.-methyl-3-oxo-1,4-androstadiene-17.beta.-carboxy acid methylester-17-propionate, ST-126.

Other medicaments produced with the process according to the invention are montelukast and pramipexol.

As adjuvants for inhalatives, especially lactose, glucose, sucrose, mannitol and/or trehalose are used.

Examples of solvent and precipitation agents, depending on the medicaments which are to be produced, are shown in the following tables, wherein solvents and precipitation agents must be miscible.

For anticholinergics/betasympathomimetics/ antiallergics:

| Active Ingredient | Solvent | Precipitating Agents |
|---|---|---|
| Salt forms | Water, methanol | Alcohols (ethanol, propanol, iso-propanol), ketones (acetone, butanone) |
| Free bases | Alcohols (ethanol, propanol, iso-propanol, tert.-butanol), ketones (acetone, butanone) | Water, methanol |

For steroids:

| Active Ingredient | Solvent | Precipitating Agents |
|---|---|---|
| Polars | Ketones (acetone, butanone) | Alcohols (methanol, ethanol) |
| | Alcohols (ethanol, propanol, iso-propanol, tert.-butanol), ketones (acetone, butanone) | Water, methanol |
| | Aromatics (toluene, ethylbenzene) | Alcohols (ethanol, propanol, iso-propanol) |
| Unpolar | Halogen hydrocarbons (dichloromethane, trichloromethane) | Alcohols (ethanol, propanol, iso-propanol), ether (dimethylether, dioxane) |

Examples of transport media are shown in the following tables, dependent on the active ingredients which are to be produced and the solvents which are used, wherein solvents and transport media are not miscible.

| Active Ingredients | Solvents | Transport Media |
|---|---|---|
| Polar | Water, alcohols (methanol, ethanol iso-propanol, tert.-butanol), ketones (acetone,, propanol, butanone) | Fluids: hydrocarbons (benzene, petrolether, cyclohexane, decaline, dodecane, benzene, toluene, xylene) Gases: air, nitrogen, carbon dioxide, helium, argon |
| Unpolar | Halogen hydrocarbons (dichloromethane, trichloromethane), ether (diethylether, dibutylether), aromatics (toluene, ethylbenzene) | Fluids water, alcohols (methanol), amides (formamide) Gases: air, nitrogen, carbon dioxide, helium, argon |

Procedures by way of examples and drawings carrying out the process according to the invention will be described in more detail hereinafter. The Examples which follow serve solely as a detailed illustration without restricting the subject matter of the invention.

### Example 1

### Continuous crystallization of inhalable fenoterol HBr using a microreactor

In order to crystallize fenoterol HBr with a particle size suitable for inhalation (90 % of all crystals are smaller than 5.8 µm) a segmented flow tubular reactor was used. Fenoterol was crystallized from water by cooling, dodecane has been used as transport medium for segmentation and formation of small water bubbles.

The following parameters must be employed in order to achieve a crystal size small enough to be suitable for inhalation:
- the starting material must be a solution with a high concentration of fenoterol in water (695 mg/ml, prepared at 90°C), which in fact represents a liquid two phase mixture
- an additive (dodecane, 6 % v./v.) needs to be added to the hot solution
- from this solution a very high supersaturation is created by rapid cooling down to 18°C
- the cooled homogeneous supersaturated solution is then allowed to rest for 22 minutes
- crystallization is induced inside small bubbles of solution by ultrasonication, the ultrasound is applied for 14 minutes
- the suspension formed is stabilized by addition of water containing a detergent (0.1 w.-% tween)

### Experimental:

The experiments were performed by dissolving 34.5 g fenoterol HBr in 50 ml of water. The solution was heated up to 90 °C in a thermostatic bath under nitrogen gas flow to dissolve the fenoterol. 3 ml of dodecane are added to the solution before the start of the experiment.

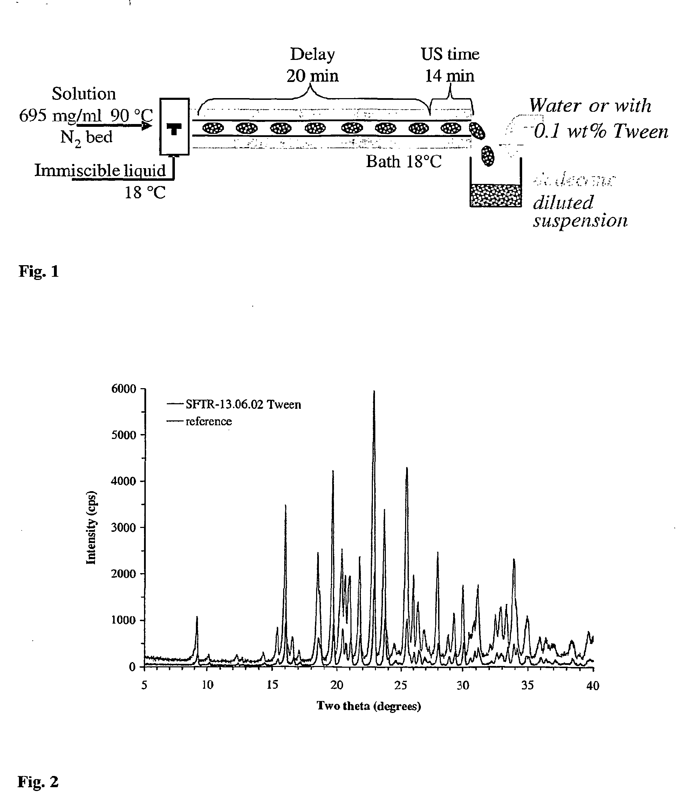

The solution is pumped through the reactor and enters the segmenter were small droplets are formed by segmentation with a transport fluid, dodecane at 18 °C. The droplets travel for 22 minutes through the tube before being treated for to 14 minutes with ultrasound. Upon ultasound treatment a highly concentrated suspension is formed inside the water phase which leaves the reactor together with the transport medium. The separation between the slurry and the transport medium was made in an open beaker to which pure water or an aqueous solution of 0.1 w.-% of tween 80 (polyethylenglycole (20) S monooleate) was added (with a (dodecane + slurry)/water ratio of about one) (see Figure 1).

### Results:

Table 1 presents the particle size distribution measured in aqueous suspension.

**Table 1: Particle size distribution data determined in suspension measured with the Malvern Mastersizer**

| **Sample** | **dᵥ₁₀(µm)** | **dᵥ₅₀(µm)** | **dᵥ₉₀(µm)** | **Span** | **Medium** |
|---|---|---|---|---|---|
| **SFTR-14.05.02** | **1.21** | **2.51** | **5.20** | **1.59** | aqueous suspension |
| **SFTR-13.06.02** | **0.77** | **1.70** | **5.92** | **3.03** | tween stabilized aqueoussuspension |

The sample was also characterized by X-ray diffraction and thermoanalysis. The powder produced by filtration and drying of the suspension was fully crystalline and conform to the starting material (figure 2).

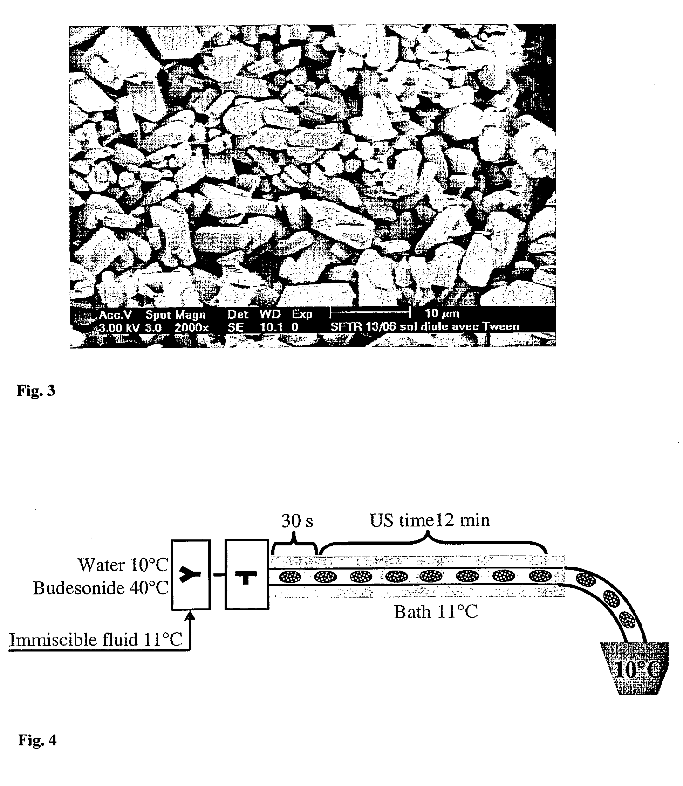

Furthermore, DSC and TGA show equivalence between starting material and crystallization product. Figure 3 shows a SEM image of the powder.

### Example 2

### Continuous crystallization of inhalable budesonide using a microreactor

Budesonide was crystallized from ethanol by a combined antisolvent and cooling crystallization using the segmented flow tubular reactor.

The following parameters must be employed in order to achieve a small crystal size:
- the starting material must be a solution with a high concentration of budesonide in ethanol (60 mg/ml, prepared at 60°C)
- an additive (hydroxy-propyl cellulose, 1 w.-%) needs to be added to the hot solution
- from this solution a very high supersaturation is created by mixing with water (1:1) as antisolvent and simultaneous cooling down to 11°C
- the cooled homogeneous supersaturated solution is then allowed to rest only for 30 seconds
- crystallization is induced inside small bubbles of solution by ultrasonication, the ultrasound is applied for 12 minutes

### Experimental:

3 g budesonide were dissolved in 50 ml of ethanol at 60°C and allowed to cool to 40 °C. 50 ml water containing 1 w.- % of hydroxy-propyl cellulose were used as antisolvent and cooled down to 10 °C.

The dodecane was saturated with ethanol prior to the experiment to avoid a diffusion of the ethanol into the dodecane phase. The dodecane was injected at the temperature of 11 °C and the thermostatic bath around the tubular section of the segmented flow tubular reactor was also maintained at 11 °C. The warm ethanolic solution of budesonide was mixed with cold antisolvent using a 2-jet mixer at a volume ratio ethanol/water of 1:1. The droplets were allowed to travel through the tube for 30 seconds before they undergo an ultrasonic treatment of 12 minutes where the tube is placed in an ultrasonic bath (cp. Figure 4). The budesonide suspension together with the transport medium were collected in a beaker maintained at 10 °C. This suspension was filtered and dried over silica gel at room temperature. The powder yield is 60%.

### Results:

The particle size distribution in the suspension produced has not been measured. Table 2 shows the particle size distribution of the dry powder re-dispersed in water containing 0.1 w.-% tween. The size distribution may be different compared to the crystals in suspension due to agglomeration during drying.

**Table 2: Particles size distribution data of budesonide powder.**

| **Sample** | **dᵥ₁₀ (µm)** | **dᵥ₅₀ (µm)** | **dᵥ₉₀ (µm)** | **Span** |
|---|---|---|---|---|
| Budesonide | 1.32 | 7.59 | 12.86 | 1.52 |

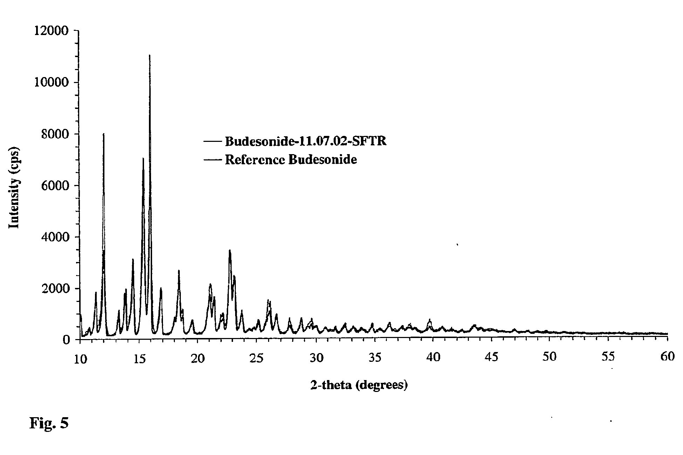

The sample was also characterized by X-ray diffraction. The powder produced by filtration and drying of the suspension was fully crystalline and conform to the starting material (figure 5).

## Claims

1. An improved process for the production of powders of inhalable medicaments by crystallization from a supersaturated fluid containing said medicament, carried out in a micro-reactor according to claim 15, the method comprising passing along a tubular reactor
(c) a segmented flow of that fluid comprised of discrete volumes; or
(d) a fluid mixture being separated by discrete volumes of a separating fluid which is substantially immiscible with said fluid,
**characterized in that** the crystallization is initiated by application of ultrasound.

2. A process as claimed in claim 1 wherein the segmented flow passes along the tubular reactor as a plug flow.

3. A process as claimed in claim 1 or 2 wherein the tubular reactor consists of the following segments:
(iv) a residence time (t_{R}) segment;
(v) an ultrasound time (t_{US}) segment; and
(vi) optionally an aging time (t_{A}) segment.

4. A process as claimed in any one of claims 1 to 3 wherein t_{US} is 1 to 30 s and t_{A} is 0.5 to 15 min.

5. A process as claimed in any one of claims 1 to 3 wherein t_{US} is 0.5 to 15 min and t_{A} is 0 to 30 s.

6. A process as claimed in any one of claims 1 to 5 wherein ultrasound with a frequency of 20 to 60 kHz is applied.

7. A process as claimed in any one of claims 1 to 6 wherein the energy density of the ultrasound applied is from 10 to 80 WL⁻¹.

8. A process as claimed in any one of claims 1 to 7 wherein the segmented flow or a precursor segmented flow from which the segmented flow is subsequently generated. is produced by passing the fluid containing the organic compound or a component thereof and the separating fluid to a chamber having a restricted outlet from which the segmented flow issues.

9. A process as claimed in any one of claims 1 to 8 wherein a fluid mixture containing the organic compound is prepared in a micro-mixer before the segmentation.

10. A process as claimed in any one of claims 1 to 9 wherein the fluid mixture is a mixture of a solution of the organic compound with a suitable precipitant to create a meta-stable supersaturated fluid.

11. A process as claimed in any one of claims 1 to 10 wherein the fluid mixture is a mixture of a solution of the organic compound with a suitable detergent in order to influence particle size and shape during the subsequent crystallization process.

12. A process as claimed in any one of claims 1 to 11 wherein the separating fluid is
a hydrocarbon, in the event that the organic compound is water-soluble; or
a lower alcohol or water, in the event that the organic compound is insoluble in water.

13. A process as claimed in claim 12 wherein the separating fluid is a C₆₋₁₈ hydrocarbon.

14. A process as claimed in any one of claims 1 to 15 wherein the organic powder is an inhalable medicament selected from the group consisting of anticholinergics, betasympathomimetics, antiallergics and steroids.

15. A micro-reactor for implementing the process according to one of the preceding claims comprising a micro-mixer, a segmented and a tubular reactor, wherein
- the dimensions of the micro-mixer for dividing the added fluids which are to be mixed is in the range of 10 µm to 1 mm, preferably between 25 µm to 200 µm,
- the dimensions of the channels of the segmenter lie in the range of 0.1 to 5 mm, preferably in the range of between 0.2 mm and 5 mm, and
- the tubular reactor is configured to be tube-, pipe- or channel-shaped with diameters of the channels in the range of 0.5 to 10 mm, preferably 1 mm to 2 mm, and with a length of between 10 cm and 200 m, preferably between 1 m and 25 m and is equipped with an external ultrasound source.

16. A micro-reactor according to claim 15, wherein the tubular reactor consists of the following segments:
(iv) a residence time (t_{R}) segment;
(v) an ultrasound time (t_{US}) segment; and
(vi) optionally an aging time (t_{A}) segment.

17. A micro-reactor according to claim 16, wherein the ultrasound time (t_{US}) segment is inserted into an ultrasound bath.

## Patentansprüche

1. Verbessertes Verfahren zur Herstellung von Pulvern von inhalierbaren Arzneimitteln durch Kristallisierung aus einer übersättigten Flüssigkeit, enthaltend das Arzneimittel, durchgeführt in einem Mikroreaktor gemäß Anspruch 15, wobei das Verfahren umfasst: das Passieren lassen eines röhrenförmigen Reaktors von
(c) einem segmentierten Strom der Flüssigkeit, umfassend diskrete Volumina; oder
(d) einer Flüssigkeitsmischung, getrennt durch diskrete Volumina einer Trennflüssigkeit, die mit der Flüssigkeit im Wesentlichen nicht mischbar ist,
**dadurch gekennzeichnet, dass** die Kristallisation durch Anwendung von Ultraschall ausgelöst wird.

2. Verfahren nach Anspruch 1, wobei der segmentierte Strom den röhrenförmigen Reaktor als ein Pfropfenstrom passiert.

3. Verfahren nach Anspruch 1 oder 2, wobei der röhrenformige Reaktor aus den folgenden Abschnitten besteht:
(iv) einem Verweilzeit-Abschnitt (t_{R});
(v) einem Ultraschallzeit-Abschnitt (t_{US}); und
(vi) gegebenenfalls einem Alterungszeit-Abschnitt (t_{A}).

4. Verfahren nach irgendeinem der Ansprüche 1 bis 3, wobei t_{US} 1 bis 30 sek, beträgt und t_{A} 0,5 bis 15 min. beträgt.

5. Verfahren nach irgendeinem der Ansprüche 1 bis 3, wobei tus 0,5 bis 15 min. beträgt und t_{A} 0 bis 30 sek. beträgt.

6. Verfahren nach irgendeinem der Ansprüche 1 bis 5, wobei Ultraschall mit einer Frequenz von 20 bis 60 kHz eingesetzt wird.

7. Verfahren nach irgendeinem der Ansprüche 1 bis 6, wobei die Energiedichte des eingesetzten Ultraschalls von 10 bis 80 WL⁻¹ beträgt.

8. Verfahren nach irgendeinem der Ansprüche 1 bis 7, wobei der segmentierte Strom oder ein segmentierter Vorläuferstrom, aus dem der segmentierte Strom nachfolgend erzeugt wird, hergestellt wird, indem die Flüssigkeit, enthaltend die organische Verbindung oder eine Komponente bzw. einen Bestandteil hiervon und die Trennflüssigkeit zu einer Kammer mit einem beschränkten bzw. gedrosselten Auslass, von dem der segmentierte Strom ausgeht, geleitet bzw. geführt wird.

9. Verfahren nach irgendeinem der Ansprüche 1 bis 8, wobei eine Flüssigkeitsmischung, enthaltend die organische Verbindung, vor der Segmentierung in einem Mikromischer hergestellt wird.

10. Verfahren nach irgendeinem der Ansprüche 1 bis 9, wobei die Flüssigkeitsmischung eine Mischung einer Lösung der organischen Verbindung mit einem geeigneten Fällmittel darstellt, um eine metastabile übersättigte Flüssigkeit herzustellen.

11. Verfahren nach irgendeinem der Ansprüche 1 bis 10, wobei die Flüssigkeitsmischung eine Mischung einer Lösung der organischen Verbindung mit einem geeigneten Detergenz bzw. oberflächenaktiven Mittel darstellt, um die Partikelgröße und -form während des nachfolgenden Kristalllsationsprozesses zu beeinflussen.

12. Verfahren nach irgendeinem der Ansprüche 1 bis 11, wobei die Trennflüssigkeit einen Kohlenwasserstoff darstellt, für den Fall, dass die organische Verbindung wasserlöslich ist; oder
einen niederen Alkohol oder Wasser darstellt, für den Fall, dass die organische Verbindung in Wasser unlöslich ist.

13. Verfahren nach Anspruch 12, wobei die Trennflüssigkeit einen C₆₋₁₈-Kohlenwasserstoff darstellt.

14. Verfahren nach irgendeinem der Ansprüche 1 bis 13, wobei das organische Pulver ein inhalierbares Arzneimittel darstellt, ausgewählt aus der Gruppe, bestehend aus Anticholinergika, Betasympathomimetika, Antiallergika und Steroiden.

15. Mikroreaktor zur Durchführung des Verfahrens nach einem der vorhergehenden Ansprüche, umfassend einen Mikromischer, einen Segmentierer und einen röhrenförmigen Reaktor, wobei
- die Dimensionen des Mikromischers zur Aufteilung der zugegebenen Flüssigkeiten, die gemischt werden sollen, im Bereich von 10 µm bis 1 mm liegen, bevorzugt zwischen 25 µm bis 200 µm,
- die Dimensionen der Kanäle des Segmentierers im Bereich von 0,1 bis 5 mm liegen, bevorzugt im Bereich zwischen 0,2 mm bis 5 mm, und
- der röhrenförmige Reaktor aufgebaut ist, dass er schlauch-, röhren- oder kanalförmig ist mit Durchmessern der Kanäle im Bereich von 0,5 bis 10 mm, bevorzugt 1 mm bis 2 mm, und einer Länge zwischen 10 cm und 200 m, bevorzugt zwischen 1 m und 25 m, und ausgestattet ist mit einer externen Ultraschallquelle.

16. Mikroreaktor nach Anspruch 15, wobei der röhrenförmige Reaktor aus den folgenden Abschnitten besteht:
(iv) einem Verweilzeit-Abschnitt (t_{R});
(v) einem Ultraschallzeit-Abschnitt (tus); und
(vi) gegebenenfalls einem Alterungszeit-Abschnitt (t_{A}).

17. Mikroreaktor nach Anspruch 16, wobei der Ultraschallzeit-Abschnitt (t_{US}) in ein Ultraschallbad eingetaucht ist.

## Revendications

1. Procédé amélioré pour la production de poudres de médicaments à inhaler par cristallisation à partir d'un fluide sursaturé contenant ledit médicament, réalisé dans un microréacteur selon la revendication 15, le procédé comprenant le passage le long d'un réacteur tubulaire
(c) d'un écoulement segmenté de ce fluide comprenant des volumes discrets ; ou
(d) d'un mélange de fluide séparé par des volumes discrets d'un fluide de séparation qui est sensiblement immiscible avec ledit fluide,
**caractérisé en ce que** la cristallisation est initiée par l'application d'ultrasons.

2. Procédé selon la revendication 1, dans lequel l'écoulement segmenté passe le long du réacteur tubulaire comme un écoulement piston.

3. Procédé selon la revendication 1 ou 2, dans lequel le réacteur tubulaire est constitué des segments suivants :
(iv) un segment de temps de séjour (t_{R}) ;
(v) un segment de temps d'ultrasons (t_{US}) ; et
(vi) éventuellement un segment de temps de vieillissement (t_{A}).

4. Procédé selon l'une quelconque des revendications 1 à 3, dans lequel t_{US} est de 1 à 30 s et t_{A} est de 0,5 à 15 min.

5. Procédé selon l'une quelconque des revendications 1 à 3, dans lequel t_{US} est de 0,5 à 15 min et t_{A} est de 0 à 30 s.

6. Procédé selon l'une quelconque des revendications 1 à 5, dans lequel des ultrasons à une fréquence de 20 à 60 kHz sont appliqués.

7. Procédé selon l'une quelconque des revendications 1 à 6, dans lequel la densité d'énergie des ultrasons appliqués est de 10 à 80 WL⁻¹.

8. Procédé selon l'une quelconque des revendications 1 à 7, dans lequel l'écoulement segmenté ou un écoulement segmenté précurseur à partir duquel l'écoulement segmenté est subséquemment généré, est produit en faisant passer le fluide contenant le composé organique ou un composant de celui-ci et le fluide de séparation jusqu'à une chambre ayant une sortie limitée d'où l'écoulement segmenté sort.

9. Procédé selon l'une quelconque des revendications 1 à 8, dans lequel un mélange de fluide contenant le composé organique est préparé dans un micro-mélangeur avant la segmentation.

10. Procédé selon l'une quelconque des revendications 1 à 9, dans lequel le mélange de fluide est un mélange d'une solution du composé organique avec un précipitant adapté pour créer un fluide sursaturé métastable.

11. Procédé selon l'une quelconque des revendications 1 à 10, dans lequel le mélange de fluide est un mélange d'une solution du composé organique avec un détergent adapté, afin d'influencer la taille et la forme de particules pendant le processus de cristallisation subséquent.

12. Procédé selon l'une quelconque des revendications 1 à 11, dans lequel le fluide de séparation est
✔ un hydrocarbure dans le cas où le composé organique est soluble dans l'eau, ou
✔ un alcool inférieur ou de l'eau, dans le cas où le composé organique est insoluble dans l'eau.

13. Procédé selon la revendication 12, dans lequel le fluide de séparation est un hydrocarbure en C₆₋₁₈.

14. Procédé selon l'une quelconque des revendications 1 à 15, dans lequel la poudre organique est un médicament à inhaler, choisi dans le groupe constitué d'anticholinergiques, de bêta-sympathomimétiques, d'agents antiallergiques et de stéroides.

15. Microréacteur pour mettre en application le procédé selon l'une des revendications précédentes, comprenant un micro-mélangeur, un segmenteur et un réacteur tubulaire, dans lequel
- les dimensions du micro-mélangeur pour diviser les fluides ajoutés qui doivent être mélangés sont dans la plage de 10 µm à 1 mm, de préférence entre 25 µm et 200 µm,
- les dimensions des canaux du segmenteur sont dans la plage de 0,1 à 5 mm, de préférence dans la plage entre 0,2 mm à 5 mm, et
- le réacteur tubulaire est configuré pour avoir la forme d'un tube, d'un conduit ou d'un canal avec des diamètres de canaux dans la plage de 0,5 à 10 mm, de préférence d'1 mm à 2 mm, et avec une longueur comprise entre 10 cm et 200 m, de préférence entre 1 m et 25 m et est équipé d'une source d'ultrasons externe.

16. Microréacteur selon la revendication 15, dans lequel le réacteur tubulaire est constitué des segments suivants :
(iv) un segment de temps de séjour (t_{R}) ;
(v) un segment de temps d'ultrasons (t_{US}), et
(vi) éventuellement un segment de temps de vieillissement (t_{A}).

17. Microréacteur selon la revendication 16, dans lequel le segment de temps d'ultrasons (t_{US}) est inséré dans un bain d'ultrasons.
